(19) Europäisches Patentamt
European Patent Office
Office européen des brevets

(11) Numéro de publication: 0 290 020 B1

(12) # FASCICULE DE BREVET EUROPEEN

(45) Date de publication de fascicule du brevet: **08.04.92** (51) Int. Cl.5: **C07C 403/00**

(21) Numéro de dépôt: **88107228.4**

(22) Date de dépôt: **05.05.88**

(54) **Nouveaux rétinoates d'ammonium quaternaires, leur utilisation en cosmétique et en dermopharmacie.**

(30) Priorité: **06.05.87 LU 86865**

(43) Date de publication de la demande: **09.11.88 Bulletin 88/45**

(45) Mention de la délivrance du brevet: **08.04.92 Bulletin 92/15**

(84) Etats contractants désignés: **AT BE CH DE ES FR GB GR IT LI NL SE**

(56) Documents cités:
**FR-A- 1 297 730**
**US-A- 2 429 171**

(73) Titulaire: **L'OREAL**
**14, Rue Royale**
**F-75008 Paris(FR)**

(72) Inventeur: **Philippe, Michel**
**3, Rue de l'Aubépine**
**F-92160 Antony(FR)**
Inventeur: **Sebag, Henri**
**26, Rue Erlanger**
**F-75016 Paris(FR)**
Inventeur: **Saint-Leger, Didier**
**55, Rue au Maire**
**F-75003 Paris(FR)**
Inventeur: **Leveque, Jean-Luc**
**45 Rue de la Tuilerie**
**F-93370 Montfermeil(FR)**

(74) Mandataire: **Casalonga, Axel et al**
**BUREAU D.A. CASALONGA - JOSSE Morassistrasse 8**
**W-8000 München 5(DE)**

EP 0 290 020 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés ammonium quaternaires et plus précisément de nouveaux rétinoates d'ammonium quaternaires, leur procédé de préparation et les compositions cosmétiques et pharmaceutiques les contenant.

On a déjà décrit dans l'état de la technique l'utilisation de différents sels d'hexadécyl triméthyl ammonium dont l'oléate comme agent germicide. (Voir en particulier Gautier et al., Bull. Soc. Chim. France (1955) (634)).

On a déjà décrit dans le brevet français n° 1.297.730, l'utilisation de sels, d'ammoniums de l'acide de vitamine A comme agents cosmétiques régulant la kératinisation de la peau.

La demanderesse vient de découvrir, ce qui fait l'objet de la présente invention, de nouveaux rétinoates d'ammonium quaternaires. Elle a constaté que ces nouveaux rétinoates d'ammonium quaternaires possédaient une activité antibactérienne vis-à-vis des souches de Propionibactérium Acnes et que ces composés pouvaient être utilisés avantageusement comme agents antiseptiques, comédolytiques, kératolytiques, verrulytiques et/ou contre diverses affections cutanées et notamment contre les dermatoses infectieuses ou non, pouvant être d'origine bactérienne, mycobactérienne et/ou liées à l'implantation de certaines levures à caractère pathogène. Ils peuvent également être utilisés comme agents antipelliculaires, ou encore pour stimuler la repousse des cheveux et freiner leur chute.

Les composés conformes à l'invention se sont également révélés avoir des propriétés antivieillissement de la peau, notamment sur le vieillissement dû au rayonnement solaire.

La demanderesse a notamment observé que ces composés sont particulièrement efficaces dans le traitement de l'acné.

L'acné, comme cela est bien connu, est un désordre cutané, polymorphe, survenant à la puberté et régressant spontanément dans la majorité des cas vers 20 à 25 ans. L'acné concerne, chez les individus touchés, toutes les zones riches en glandes sébacées (le front, la face, les ailes du nez, le torse, le dos) à l'exception du cuir chevelu.

L'étiopathogénie de l'acné, bien que mal définie, prend son origine dans la formation d'une lésion caractéristique, le comédon. Celui-ci résulte de l'obstruction du canal pilosébacé par suite d'une dyskératinisation de la zone de l'infundibulum du canal.

Cette obstruction a pour effet majeur de modifier la rhéologie du sébum et les caractéristiques physicochimiques du milieu, tels que pH, tension de vapeur d'oxygène, etc...

Cette modification permet l'hyperprolifération des souches résidentes cutanées, principalement le Propionibactérium Acnes, qui est une souche anaérobie ou aéro-tolérante.

L'hyperprolifération bactérienne a pour conséquence de libérer dans le milieu certaines protéases ou hyaluronidases, d'origine bactérienne, qui provoquent une lyse du sac folliculaire et ainsi la libération des composés inflammatoires au sein du derme et déclenchent la réaction de type inflammatoire de l'organisme.

Si la nature des composés inflammatoires est à l'heure actuelle non déterminée, leur origine bactérienne semble faire peu de doute, expliquant le bon succès thérapeutique dans l'acné inflammatoire de composés antibactériens tant par voie orale que par voie topique.

On a préconisé, dans ce but, très souvent, des agents antibactériens, comme les antibiotiques tels que l'érythromycine, la tétracycline, la clindamycine, dans le traitement de l'acné. Ces composés donnent des résultats excellents, mais leur utilisation trop poussée rend les souches de Propionibactérium Acnes résistantes à ces mêmes antibiotiques, de telle sorte que le traitement répété peut s'avérer peu efficace.

La demanderesse a découvert que les nouveaux rétinoates d'ammonium quaternaires possèdent une activité antibactérienne "in vitro" vis-à-vis de Propionibactérium Acnes proche de celle des antibiotiques utilisés par voie topique contre l'acné comme notamment l'érythromycine et la clindamycine, sans présenter les limitations de ces derniers dus à une utilisation répétée.

La demanderesse a également constaté que les nouveaux rétinoates d'ammonium quaternaires ont une activité comédolytique et kératolytique supérieure à celle de l'oléate d'hexadécyl triméthyl ammonium.

L'invention a donc pour objet, à titre de composés nouveaux, des rétinoates d'ammonium quaternaires définis ci-après.

Un autre objet de l'invention consiste en des compositions contenant les composés définis ci-dessus et utilisables dans des traitements thérapeutiques.

L'invention a également pour objet une composition et un procédé de traitement cosmétique à base de ces composés.

D'autres objets de l'invention apparaîtront à la lecture de la description et des exemples qui suivent.

Les rétinoates d'ammonium quaternaires qui constituent l'objet principal de l'invention sont des

composés répondant essentiellement à la formule (I) suivante.

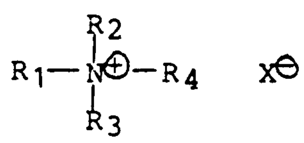

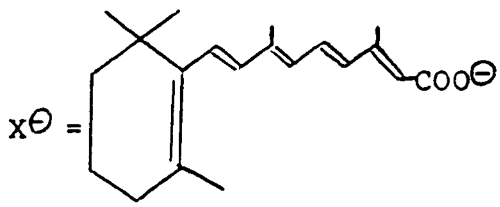

ou,

rétinoate all trans

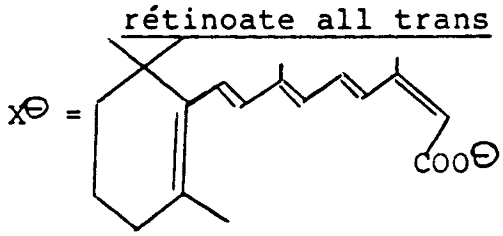

rétinoate 13 cis

dans laquelle :

(i) $R_1$, $R_2$, $R_3$, identiques ou différents, désignent un groupement alcoyle linéaire saturé en $C_1$-$C_4$ pouvant porter en bout de chaîne ou dans la chaîne un ou plusieurs groupements hydroxyles; $R_4$ désigne un groupement alcoyle ou alcényle linéaire en $C_{12}$-$C_{18}$;

(ii) $R_3$ désigne le groupement :

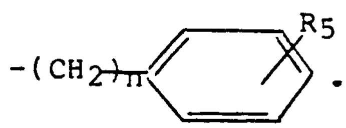

dans lequel n est égal à 0 ou 1, $R_5$ représente un atome d'hydrogène, un hydroxyle, un atome d'halogène, un groupement alcoyle ou hydroxyalcoyle en $C_1$ à $C_{18}$ ou un groupement acyle en $C_2$ à $C_{18}$, $R_1$, $R_2$ et $R_4$ ayant les significations indiquées ci-dessus;

(iii) $R_1$ et $R_2$ peuvent former un hétérocycle aliphatique comportant éventuellement un atome d'oxygène, un autre atome d'azote ou un atome de soufre, $R_3$ et $R_4$ ayant les significations définies ci-dessus en (i) et (ii).

Les composés conformes à l'invention sont préparés de préférence à partir d'un sel tel que plus particulièrement les carbonates d'ammonium quaternaires correspondants solubilisés dans un solvant tel que le méthanol, l'éthanol, l'isopropanol, le tetrahydrofuranne, le dioxanne ou leur mélange auquel on ajoute l'acide rétinoïque choisi, solubilisé dans un de ces solvants précités. La réaction est réalisée de préférence sous un courant de gaz inerte, tel que l'azote ou l'argon.

Les compositions, objet de l'invention, sont essentiellement caractérisées par le fait qu'il s'agit de compositions topiques contenant dans un support approprié pour une application topique, au moins l'un des composés répondant à la formule (I) ci-dessus.

Les compositions peuvent se présenter sous forme de solution, d'émulsion, de suspension, de gel ou de dispersion vésiculaire, contenant au moins un composé répondant à la formule (I) dans des concentrations comprises entre 0,00001 et 1% en poids par rapport au poids total de la composition, et de préférence comprises entre 0,001 et 1%.

Les compositions peuvent contenir des véhicules et adjuvants, acceptables pour une application topique, bien connus dans l'état de la technique. On peut par exemple préparer des solutions en utilisant un ou plusieurs solvant(s) organique(s) acceptable(s) du point de vue physiologique, choisi(s), en plus de l'eau, parmi les solvants tels que l'acétone, l'éthanol, l'alcool isopropylique, les éthers de glycols, tels que les produits vendus sous la dénomination de "Dowanol"®, les polyglycols, tels que les polyéthylèneglycols, polypropylèneglycols, les esters d'acides organiques à courte chaîne, tels que, de préférence, les lactates d'alkyle en $C_1$-$C_4$, les esters d'acides gras, tels que, par exemple, le myristate d'isopropyle, les triglycérides d'origine naturelle ou synthétique, les huiles et cires minérales.

Les compositions conformes à l'invention peuvent également renfermer des agents tensio-actifs

ioniques et/ou de préférence non ioniques, des polymères ioniques et/ou de préférence non ioniques, d'origine naturelle ou synthétique, en particulier les protéines, la cellulose et/ou les dérivés de la cellulose, la gomme de guar, de caroube, la gomme arabique, des biohétéropolysaccharides tels que la gomme de xanthane, la chitine, le chitosane ou ses dérivés, ou bien des bentones et des montmorillonites, etc...

On peut ajouter, si nécessaire, un adjuvant choisi parmi les agents anti-oxydants, les tensio-actifs, d'autres agents conservateurs, des kératolytiques ou comédolytiques, des parfums et des colorants.

Par exemple, parmi les anti-oxydants, on peut citer la t-butylhydroxyquinone, le butylhydroxyanisole, le butylhydroxytoluène et l' $\alpha$-tocophérol et ses dérivés.

Les formes galéniques principalement conditionnées pour la voie topique, se présentent sous forme de crèmes, de laits, de gels, de dispersions ou microémulsions, de vésicules, de lotions plus ou moins épaissies, de tampons imbibés, de pommades, de sticks ou bien sous forme de formulations aérosols en forme de spray ou de mousse, ou encore sous forme de pain de savon.

Les compositions conformes à l'invention sont particulièrement appropriées pour le traitement de diverses dermatoses telle que l'acné. Dans ce cas, elles peuvent également contenir, dans un milieu pharmaceutiquement acceptable, en association avec le composé de formule (I), des agents antiacnéiques tels que les agents antibactériens, les anti-inflammatoires et les composés antiséborrhéïques.

Les composés conformes à l'invention, lorsqu'ils sont utilisés pour traiter la chute des cheveux ou pour stimuler leur repousse, peuvent être associés à des agents favorisant la repousse et plus particulièrement les dérivés de pyrimidine tel que le diamino-2,4 pipéridino-6 pyrimidine oxyde-3 encore appelé MINOXIDIL, le chloro-7 méthyl-3 benzothiadiazine-1,2,4 dioxyde-1,1 ou "DIAZOXIDE", le diphényl-5,5 imidazolidine dione-2,4 ou "PHENYTOIN".

Un objet de l'invention réside dans l'utilisation des composés de formule (I) pour la préparation de telles compositions destinées au traitement des dermatoses.

Les compositions conformes à l'invention peuvent également être utilisées pour le traitement cosmétique de la peau, notamment comme agents comédolytiques et kératolytiques.

Ces compositions peuvent contenir, dans un milieu cosmétiquement acceptable, des adjuvants habituellement utilisés en cosmétique dans des compositions destinées aux soins et au traitement cosmétique de la peau.

La méthode de traitement cosmétique consiste à appliquer une telle composition sur la peau pendant une période de 2 à 6 mois.

L'activité antibactérienne des composés conformes à l'invention a été étudiée par la méthode de dilution en vue de déterminer la Concentration Minimale Inhibitrice (C.M.I.) suivant la méthode décrite et employée par G.A. DENYS et al., Antimicrobial Agents and Chemotherapy (1983) 23, 335-337, et J.J. LEYDEN et al., J. Am. Acad. Dermatol. (1983) 8 (1) 41-5, en utilisant comme souche de Propionibactérium Acnes, la souche ATCC 6919.

Les concentrations minimales inhibitrices (C.M.I.) exprimées en $\mu$g/ml (DMSO) des rétinoates d'ammonium quaternaires, conformes à l'invention, figurent dans le tableau I.

| TABLEAU I = TESTS C.M.I. | | | | | | | * témoins |
|---|---|---|---|---|---|---|---|
| N° | NOM DU COMPOSE | C.M.I. (µg/ml) ATCC 6919 | $R_1$ | $R_2$ | $R_3$ | $R_4$ | CONTRE ANION |
| 1* | Oléate de N-hexadécyl N,N,N-triméthylammonium | 12 | $-CH_3$ | $-CH_3$ | $-CH_3$ | $n-C_{16}H_{33}$ | Oléate |
| 2 | Rétinoate all trans de N-hexadécyl N,N,N-triméthylammonium | 2,2 | $-CH_3$ | $-CH_3$ | $-CH_3$ | $n-C_{16}H_{33}$ | Rétinoate all trans |
| 3 | Rétinoate 13-cis de N-hexadécyl N,N,N-triméthylammonium | 3 | $-CH_3$ | $-CH_3$ | $-CH_3$ | $n-C_{16}H_{33}$ | Rétinoate 13-cis |
| 4 | Rétinoate all trans de N-benzyl N,N-diméthyl N-hexadécylammonium | 0,6 | $-CH_3$ | $-CH_3$ | 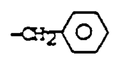 | $n-C_{16}H_{33}$ | Rétinoate all trans |
| 5 | Rétinoate 13-cis de N-benzyl N,N-diméthyl N-hexadécylammonium | 1,1 | $-CH_3$ | $-CH_3$ | 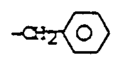 | $n-C_{16}H_{33}$ | Rétinoate 13-cis |
| 6 | Rétinoate all trans de N-hexadécyl N,N-diméthyl N,ß-hydroxyéthylammonium | 1,4 | $-CH_3$ | $-CH_3$ | $-C_2H_4OH$ | $n-C_{16}H_{33}$ | Rétinoate all trans |
| 7 | Rétinoate all trans de N-dodécyl N-éthyl N,N-diméthylammonium | 0,8 | $-CH_3$ | $-CH_3$ | $-C_2H_5$ | $n-C_{12}H_{25}$ | Rétinoate all trans |
| 8 | Rétinoate all trans de N-méthyl N-dodécyl morpholinium | 3,5 | 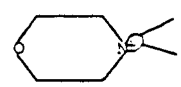 | | $-CH_3$ | $n-C_{12}H_{25}$ | Rétinoate all trans |
| 9 | Rétinoate all trans de N-dodécyl N,N,N-triméthylammonium | 3 | $-CH_3$ | $-CH_3$ | $-CH_3$ | $n-C_{12}H_{25}$ | Rétinoate all trans |

Il apparaît à l'étude de ce tableau que les rétinoates d'ammonium quaternaires (all trans et 13-cis) conformes à l'invention sont nettement plus actifs sur la souche de Propionibactérium Acnes que l'oléate d'hexadécyltriméthylammonium pris comme substance de référence.

Les exemples ci-après sont destinés à illustrer, d'une part, les procédés de préparation des composés conformes à l'invention, et, d'autre part, des compositions pharmaceutiques et cosmétiques mettant en oeuvre ces composés.

EXEMPLE DE PREPARATION 1

Préparation du rétinoate all trans de N-hexadécyl N,N, N-triméthylammonium : composé n°2 (tableau I).

A une solution de 5,25 g (8,3 mmoles) de carbonate d'hexadécyltriméthylammonium fraîchement purifié et séché, dissous dans 50 ml de méthanol anhydre, sont ajoutés 5 g (16,6 mmoles) d'acide rétinoïque all trans dissous préalablement dans 50 ml de tétrahydrofuranne anhydre; le mélange est agité 2 heures à 30 ° C à l'abri de la lumière et sous atmosphère inerte, puis les solvants sont évaporés sous un vide poussé et le résidu obtenu est repris dans l'heptane; la solution est filtrée et le solvant est évaporé sous un vide poussé. On obtient 9,7 g (rendement quantitatif) de rétinoate all trans de N-hexadécyl N,N,N-triméthylammonium sous forme de pâte marron.

| Analyse élémentaire : $C_{39}H_{69}NO_2,1H_2O$; M = 602 | | | |
|---|---|---|---|
| | C | H | N |
| Calculé % : | 77,82 | 11,89 | 2,32 |
| Trouvé % : | 77,44 | 12,04 | 2,00 |

R.M.N. $^{13}C$ et $^{1}H$ ($CDCl_3$, réf. interne T.M.S.)

Les spectres de R.M.N. du $^{1}H$ et du $^{13}C$ confirment la structure attendue; notamment le spectre de R.M.N. du $^{13}C$ indique un déplacement vers les champs faibles du C-14 (+7,6 ppm) dû à la formation du rétinoate de l'ammonium quaternaire; enfin, le spectre de R.M.N. du $^{1}H$ précise que la stéréochimie de la chaîne rétinoïque est all trans grâce au déplacement chimique du H-12 à 6,3 ppm.
Spectre U.V. (éthanol) $\lambda$ max = 336 nm $\epsilon$mol = 41579.

EXEMPLE DE PREPARATION 2

Préparation du rétinoate 13-cis de N-hexadécyl N,N,N-triméthylammonium : composé n°3 (tableau I).

A une solution de 5,25 g (8,3 mmoles) de carbonate d'hexadécyltriméthylammonium fraîchement purifié et séché, dissous dans 50 ml de méthanol anhydre, sont ajoutés 5 g (16,6 mmoles) d'acide rétinoïque 13-cis dissous préalablement dans 50 ml de tétrahydrofuranne anhydre; le mélange est agité 2 heures à 30°C à l'abri de la lumière et sous atmosphère inerte, puis les solvants sont évaporés sous un vide poussé et le résidu obtenu est repris dans l'heptane; la solution est filtrée et le solvant est évaporé sous un vide poussé. On obtient 9,7 g (rendement quantitatif) de rétinoate 13-cis de N-hexadécyl N,N,N-triméthylammonium sous forme de pâte brune.

| Analyse élémentaire : $C_{39}H_{69}NO_2$, 1,5 $H_2O$; M = 611 | | | |
|---|---|---|---|
| | C | H | N |
| Calculé % : | 76,67 | 11,54 | 2,29 |
| Trouvé % : | 77,06 | 11,68 | 2,21 |

R.M.N. $^{1}H$ et $^{13}C$ ($CDCl_3$, réf. interne T.M.S.)

Le spectre de R.M.N. $^{1}H$ confirme la structure 13-cis de la chaîne rétinoate avec le déplacement chimique du $H_{12}$ à 7,9 ppm; le spectre de R.M.N. du $^{13}C$ indique un déplacement vers les champs faibles du $C_{14}$ (+9 ppm) dû à la formation du contre anion rétinoate.
Spectre U.V. (éthanol) $\lambda$ max = 339 nm $\epsilon$mol = 30600.

EXEMPLE DE PREPARATION 3

Préparation du rétinoate all trans de N-benzyl N,N-diméthyl N-hexadécylammonium : composé n°4 (tableau I).

A une solution de 6,5 g (8,3 mmoles) de carbonate de benzyldiméthylhexadécylammonium fraîchement purifié et séché, dissous dans 50 ml de méthanol anhydre, sont ajoutés 5 g (16,6 mmoles) d'acide rétinoïque all trans dissous préalablement dans 50 ml de tétrahydrofuranne anhydre; le mélange est agité 2 heures à 30°C à l'abri de la lumière et sous atmosphère inerte, puis les solvants sont évaporés sous un vide poussé et le résidu obtenu est repris dans l'heptane; la solution est filtrée et le solvant est évaporé sous un vide poussé. On obtient 10,9 g (rendement quantitatif) de rétinoate all trans de N-benzyl N,N-diméthyl N-hexadécylammonium sous forme de pâte brune.

| Analyse élémentaire : $C_{45}H_{73}NO_2$, $H_2O$; M = 678 | | | |
|---|---|---|---|
| | C | H | N |
| Calculé % : | 79,77 | 11,07 | 2,06 |
| Trouvé % : | 79,79 | 11,11 | 2,1 |

R.M.N. du $^{13}C$ ($CDCl_3$, réf. interne T.M.S.)

Déplacement vers les champs faibles de 7,3 ppm pour le $C_{14}$ indiquant la formation du contre anion rétinoate d'ammonium quaternaire; pic du $C_{20}$ à 13,68 ppm confirmant la stéréochimie all trans de la chaîne rétinoïque; pic des méthyles de l'ammonium à 49,64 ppm.
Spectre U.V. (éthanol) $\lambda$ max = 335 $\epsilon$mol = 30378.

EXEMPLE DE PREPARATION 4

Préparation du rétinoate 13-cis de N-benzyl N,N-diméthyl N-hexadécylammonium : composé n°5 (tableau I).

A une solution de 6,5 g (8,3 mmoles) de carbonate de benzyldiméthylhexadécylammonium fraîchement purifié et séché, dissous dans 50 ml de méthanol anhydre, sont ajoutés 5 g (16,6 mmoles) d'acide rétinoïque 13-cis dissous préalablement dans 50 ml de tétrahydrofuranne anhydre; le mélange est agité 2 heures à 30°C à l'abri de la lumière et sous atmosphère inerte, puis les solvants sont évaporés sous un vide poussé et le résidu obtenu est repris dans l'heptane; la solution est filtrée et le solvant est évaporé sous un vide poussé. On obtient 10,9 g (rendement quantitatif) de rétinoate 13-cis de N-benzyl N,N-diméthyl N-hexadécylammonium sous forme de pâte marron.

| Analyse élémentaire : $C_{45}H_{73}NO_2$, $H_2O$; M = 678 | | | |
|---|---|---|---|
| | C | H | N |
| Calculé % :<br>Trouvé % : | 79,77<br>79,38 | 11,07<br>11,14 | 2,06<br>1,98 |

R.M.N. du $^{13}C$ ($CDCl_3$, réf. interne T.M.S.)

Déplacement vers les champs faibles de 9 ppm pour le $C_{-14}$ indiquant la formation du contre anion rétinoate d'ammonium quaternaire; pic du $C_{-20}$ à 20,41 ppm confirmant la stéréochimie 13-cis de la chaîne rétinoïque; pic des méthyles de l'ammonium à 49,56 ppm.
Spectre U.V. (éthanol) $\lambda$ max = 336 $\epsilon$mol = 37772.

EXEMPLE DE PREPARATION 5

Préparation du rétinoate all trans de N,N-diméthyl N-hexadécyl N-$\beta$-hydroxyéthylammonium : composé n°6(tableau I).

A une solution de 5,75 g (8,3 mmoles) de carbonate de diméthyl hexadécyl $\beta$-hydroxyéthylammonium fraîchement purifié et séché, dissous dans 50 ml de méthanol anhydre, sont ajoutés 5 g (16,6 mmoles) d'acide rétinoïque all trans dissous préalablement dans 50 ml de tétrahydrofuranne anhydre; le mélange est agité 4 heures à 30°C à l'abri de la lumière et sous atmosphère inerte, puis les solvants sont évaporés sous un vide poussé et le résidu obtenu est repris dans l'acétate d'éthyle; la solution est filtrée et le solvant est évaporé sous un vide poussé. On obtient 10 g (rendement quantitatif) de rétinoate all trans de N,N-diméthyl N-$\beta$-hydroxyéthyl N-hexadécylammonium sous forme de pâte brune.

| Analyse élémentaire : $C_{40}H_{71}NO_3$,$H_2O$; M = 632 | | | |
|---|---|---|---|
| | C | H | N |
| Calculé % :<br>Trouvé % : | 75,9<br>75,54 | 11,55<br>11,53 | 2,21<br>1,92 |

R.M.N. du $^{1}H$ ($CDCl_3$, réf. interne T.M.S.)

Déplacement vers les champs faibles de 7,8 ppm du $C_{-14}$ indiquant la formation du contre anion rétinoate d'ammonium quaternaire; pic du $C_{-20}$ à 13,67 ppm confirmant la stéréochimie all trans de la

chaîne rétinoïque.
Spectre U.V. (éthanol) λ max = 328 εmol = 31051.

EXEMPLE DE PREPARATION 6

Préparation du rétinoate all trans de N-dodécyl N-éthyl N,N-diméthylammonium : composé n°7 (tableau I).

Ce composé est préparé selon le mode opératoire décrit dans les exemples précédents.

| Analyse élémentaire : $C_{36}H_{63}NO_2,2H_2O$; M = 577,9 | | | |
|---|---|---|---|
| | C | H | N |
| Calculé % :<br>Trouvé % : | 74,8<br>75,1 | 11,68<br>11,34 | 2,42<br>2,08 |

R.M.N. du $^{13}C$ ($CDCl_3$, réf. interne T.M.S.)

Déplacement vers les champs faibles de 7,7 ppm du $C_{14}$ indiquant la formation du contre anion rétinoate d'ammonium quaternaire; pic du $C_{20}$ à 13,66 ppm confirmant la stéréochimie all trans de la chaîne rétinoïque; pic des méthyles portés par l'ammonium à 50,46 ppm.
Spectre U.V. (éthanol) λ max = 336 εmol = 38625.

EXEMPLE DE PREPARATION 7

Préparation du rétinoate all trans de N-dodécyl N-méthyl morpholinium : composé n°8 (tableau I).

Ce composé est préparé selon le mode opératoire décrit dans les exemples précédents.

| Analyse élémentaire : $C_{37}H_{63}NO_3;0,5H_2O$; M = 578,9 | | | |
|---|---|---|---|
| | C | H | N |
| Calculé % :<br>Trouvé % : | 76,76<br>76,62 | 11,14<br>10,95 | 2,42<br>2,20 |

R.M.N. du $_{13}C$ ($CDCl_3$, réf. interne T.M.S.)

Déplacement vers les champs faibles de 7,9 ppm du $C_{14}$ indiquant la formation du contre anion rétinoate d'ammonium quaternaire; pic du $C_{20}$ à 13,78 ppm; pic du méthyle porté par l'ammonium à 46,94 ppm.
Spectre U.V. (éthanol) λ max = 336 εmol = 38416.

EXEMPLE DE PREPARATION 8

Préparation du rétinoate all trans de N-dodécyl N,N,N-triméthylammonium : composé n°9 (tableau I).

Ce composé est préparé selon le mode opératoire décrit dans les exemples précédents.

| Analyse élémentaire : $C_{35}H_{61}NO_2,H_2O$; M = 545,9 | | | |
|---|---|---|---|
| | C | H | N |
| Calculé % :<br>Trouvé % : | 77,1<br>77,11 | 11,63<br>11,49 | 2,56<br>2,28 |

R.M.N. du $^{13}C$ ($CDCl_3$, réf. interne T.M.S.)

Déplacement vers les champs faibles de 7,5 ppm du $C_{-14}$ indiquant la formation du contre anion rétinoate d'ammonium quaternaire; pic du $C_{-20}$ à 13,64 ppm; pic des méthyles portés par l'ammonium à 52,97 ppm.

EXEMPLES DE COMPOSITIONS

On prépare les compositions antiacnéiques suivantes :

EXEMPLE 1

- Hydroxypropylcellulose 1,5 g
- Butylhydroxytoluène 0,05 g
- Rétinoate all trans d'hexadécyltriméthylammonium 0,03 g
- Isopropanol anhydre qsp 100 g

Cette composition se présente sous forme de gel.

EXEMPLE 2

- Hydroxypropylcellulose 1,5 g
- Butylhydroxytoluène 0,05 g
- Rétinoate 13-cis d'hexadécyltriméthylammonium 0,02 g
- Ethanol anhydre qsp 100g

Cette composition se présente sous forme de gel. EXEMPLE 3

- Hydroxypropylcellulose 1,5 g
- Butylhydroxytoluène 0,04 g
- Triglycérides d'acides gras en $C_8$-$C_{12}$ 10,00g
- Rétinoate all trans de diméthylbenzylhexadécylammonium 0,025 g
- Isopropanol anhydre qsp 100 g

Cette composition se présente sous forme de gel.

EXEMPLE 4

- Hydroxypropylcellulose 1,5 g
- Butylhydroxytoluène 0,05 g
- Lactate d'éthyle 10,00 g
- Rétinoate 13-cis de diméthylbenzylhexadécylammonium 0,03 g
- Isopropanol anhydre qsp 100 g

Cette composition se présente sous forme de gel.

EXEMPLE 5

- Rétinoate all trans d'hexadécyltriméthylammonium 0,1 g
- Butylhydroxytoluène 0,05 g
- Isopropanol anhydre qsp 100 g

Cette composition se présente sous forme de lotion.

EXEMPLE 6

- Rétinoate 13-cis d'hexadécyltriméthylammonium 0,15 g
- Butylhydroxytoluène 0,05 g
- Ethanol anhydre qsp 100 g

Cette composition se présente sous forme de lotion.

EXEMPLE 7

- Triglycérides d'acides gras en $C_8$-$C_{12}$ 15,00 g

- Rétinoate all trans dodécyltriméthylammonium 0,20 g
- Butylhydroxytoluène 0,05 g
- Isopropanol anhydre qsp 100 g

Cette composition se présente sous forme de lotion.

EXEMPLE 8

- Vaseline blanche 50,00 g
- Huile de vaseline 15,00 g
- Paraffine raffinée 34,93 g
- Rétinoate all trans d'hexadécyltriméthylammonium 0,02 g
- Butylhydroxytoluène 0,05 g

Cette composition se présente sous forme de stick.

Mode d'application : les formulations précédentes peuvent être appliquées chaque soir sur les zones à traiter pendant une période de 2 à 6 mois.

On observe une regression de l'acné après quelques semaines de traitement.

EXEMPLE 9

On prépare la lotion verrulytique suivante :

- Rétinoate all-trans d'hexadécyltriméthylammonium 0,1 g
- Butylhydroxytoluène 0,05 g
- Isopropanol anhydre qsp 100 g

Cette solution est appliquée une fois par jour sur les verrues pendant une période de 2 à 4 semaines.

EXEMPLE 10

On prépare la lotion antipelliculaire suivante :

- Rétinoate all-trans de benzyl triméthylammonium 0,001 g
- Butylhydroxytoluène 0,05 g
- Isopropanol anhydre qsp 100 g

Cette lotion est appliquée sur le cuir chevelu une fois par jour pendant 15 jours et ensuite deux fois par semaine pendant 1 mois.

EXEMPLE 11

On prépare la lotion suivante, active contre la dermatite séborréïque :

- Rétinoate 13-cis d'hexadecyl triméthylammonium 0,005 g
- Butylhydroxytoluène 0,05 g
- Isopropanol anhydre qsp 100 g

Cette lotion est appliqué sur les zones à traiter une fois par jour pendant une période de 3 à 4 semaines.

EXEMPLE 12

On prépare la lotion suivante, active contre le psoriasis :

- Rétinoate all-trans d'hexadécyl triméthylammonium 0,001 g
- Butylhydroxytoluène 0,05 g
- Isopropanol anhydre qsp 100 g

Cette composition est appliquée sur les zones à traiter une ou deux fois par jour pendant 15 jours.

**Revendications**

**Revendications pour les Etats contractants suivants : AT, BE, CH, DE, FR, GB, IT, LI, NL, SE**

**1.** Rétinoate d'ammonium quaternaire caractérisé par le fait qu'il répond à la formule :

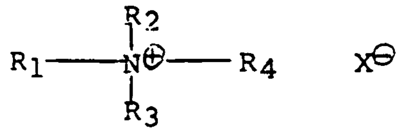

dans laquelle :

$X^{\ominus}$ désigne :

- soit un rétinoate all trans de formule :

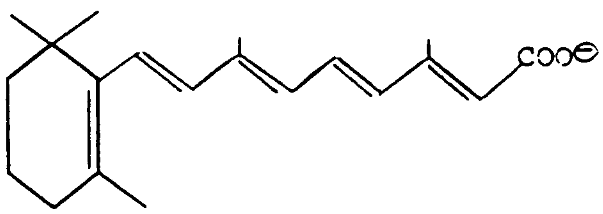

- soit un rétinoate 13-cis de formule :

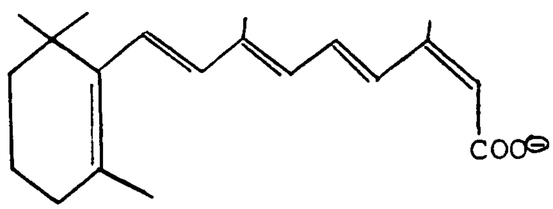

(i) $R_1$, $R_2$, $R_3$, identiques ou différents, désignent un groupement alcoyle linéaire saturé en $C_1$-$C_4$ pouvant porter en bout de chaîne ou dans la chaîne un ou plusieurs groupement(s) hydroxyle(s);

$R_4$ désigne un groupement alcoyle ou alcényle linéaire en $C_{12}$-$C_{18}$;

(ii) $R_3$ désigne le groupement :

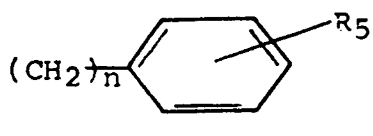

dans lequel n est égal à 0 ou 1, $R_5$ représente un atome d'hydrogène, un hydroxyle, un atome d'halogène, un groupement alcoyle ou un hydroxyalcoyle en $C_1$ à $C_{18}$ ou un groupement acyle en $C_2$ à $C_{18}$;

$R_1$, $R_2$ et $R_4$ ayant les mêmes significations que celles sous (i);

(iii) $R_1$ et $R_2$ peuvent former un hétérocycle aliphatique comportant éventuellement un atome d'oxygène, un autre atome d'azote ou un atome de soufre;

$R_3$ et $R_4$ ayant les mêmes significations que celles sous (i) et (ii).

**2.** Composés selon la revendication 1, caractérisés par le fait que $R_3$ désigne un groupement benzyle et $R_1$, $R_2$, $R_4$ ont les significations indiquées en (i) et (ii).

**3.** Composés selon l'une quelconque des revendications 1 ou 2, caractérisés par le fait qu'ils sont choisis parmi les composés suivants : rétinoate all trans de N-hexadécyl N,N,N-triméthylammonium, rétinoate 13-cis de N-hexadécyl N,N,N-triméthylammonium, rétinoate all trans de N-benzyl N,N-diméthyl N-hexadécylammonium, rétinoate 13-cis de N-benzyl N,N-diméthyl N-hexadécylammonium, rétinoate all trans de N-hexadécyl N,N-diméthyl N,$\beta$-hydroxyéthylammonium, rétinoate all trans de N-dodécyl N-éthyl N,N-diméthylammonium, rétinoate all trans de N-méthyl, N-dodécylmorpholinium, rétinoate all trans de N-dodécyl N,N,N-triméthylammonium.

**4.** Composition caractérisée par le fait qu'elle contient dans un support approprié pour une application topique, au moins un composé tel que défini dans l'une quelconque des revendications 1 à 3.

**5.** Composition selon la revendication 4, caractérisée par le fait qu'elle se présente sous forme de solution, d'émulsion, de suspension, de gel ou de dispersion vésiculaire contenant au moins un composé de formule (I) dans des concentrations comprises entre 0,00001 et 1% en poids par rapport au poids total de la composition.

**6.** Composition selon l'une quelconque des revendications 4 ou 5, caractérisée par le fait qu'elle se présente sous forme de crèmes, de laits, de gels de dispersions ou micro-émulsions, de lotions plus ou moins épaissies, de tampons imbibés, de pommades, de sticks ou sous forme de spray ou de mousse, ou bien encore sous forme de pain de savon.

**7.** Composition selon l'une quelconque des revendications 4 à 6, caractérisée par le fait qu'elle contient en association avec le composé de formule (I) au moins un autre agent antiacnéïque choisi parmi les antibactériens, les anti-inflammatoires et les composés antiséborrhéïques.

**8.** Composition selon l'une quelconque des revendications 4 à 7, caractérisée par le fait qu'elle contient en plus du composé de formule (I), le diamino-2,4 pipéridino-6 pyrimidine oxyde-3, le chloro-7 méthyl-3 benzothiadiazine-1,2,4 dioxyde-1,1, le diphényl-5,5 imidazolidine dione-2,4.

**9.** Composition selon l'une quelconque des revendications 4 à 8 pour son application thérapeutique.

**10.** Utilisation du composé défini dans l'une quelconque des revendications 1 à 3, pour la préparation d'une composition destinée au traitement des affections cutanées.

**11.** Utilisation selon la revendication 8, caractérisée par le fait que l'on prépare une composition destinée au traitement de l'acné.

**12.** Utilisation du composé selon l'une quelconque des revendications 1 à 3 pour la préparation d'un produit stimulant la repousse et/ou freinant la chute des cheveux.

**13.** Méthode de traitement cosmétique caractérisée par le fait qu'elle consiste à appliquer sur la peau une composition selon l'une quelconque des revendications 4 à 8.

**Revendications pour les Etats contractants suivants : ES, GR**

**1.** Procédé de préparation d'un rétinoate d'ammonium quaternaire répondant à la formule (I) :

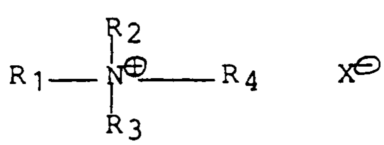

dans laquelle :
$X^{\ominus}$ désigne :
- soit un rétinoate all trans de formule :

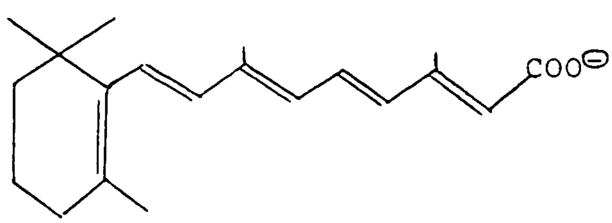

- soit un rétinoate 13-cis de formule :

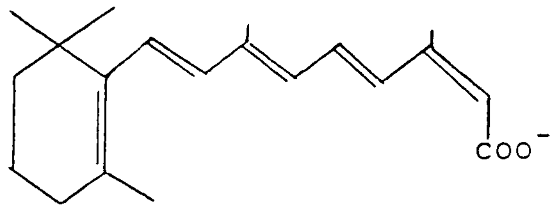

(i) $R_1$, $R_2$, $R_3$, identiques ou différents, désignent un groupement alcoyle linéaire saturé en $C_1$-$C_4$ pouvant porter en bout de chaîne ou dans la chaîne un ou plusieurs groupement(s) hydroxyle(s);

$R_4$ désigne un groupement alcoyle ou alcényle linéaire en $C_{12}$-$C_{18}$;

(ii) $R_3$ désigne le groupement :

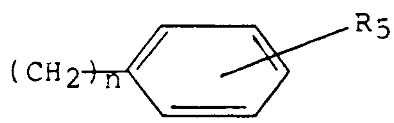

dans lequel n est égal à 0 ou 1, $R_5$ représente un atome d'hydrogène, un hydroxyle, un atome d'halogène, un groupement alcoyle ou un hydroxyalcoyle en $C_1$ à $C_{18}$ ou un groupement acyle en $C_2$ à $C_{18}$;

$R_1$, $R_2$ et $R_4$ ayant les mêmes significations que celles sous (i);

(iii) $R_1$ et $R_2$ peuvent former un hétérocycle aliphatique comportant éventuellement un atome d'oxygène, un autre atome d'azote ou un atome de soufre;

$R_3$ et $R_4$ ayant les mêmes significations que celles sous (i) et (ii);

caractérisé par le fait que l'on fait réagir un sel d'ammonium quaternaire répondant à la formule (Ia) :

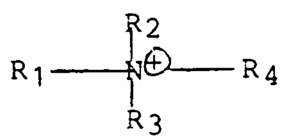

(Ia)

dans laquelle $R_1$, $R_2$, $R_3$ et $R_4$ ont les mêmes significations que celles définies dans la formule (I), solubilisé dans un solvant avec

- soit un acide rétinoïque all-trans de formule (Ib) :

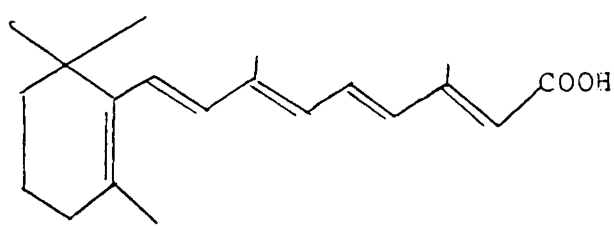

(Ib)

- soit un acide rétinoïque 13-cis de formule (Ic) :

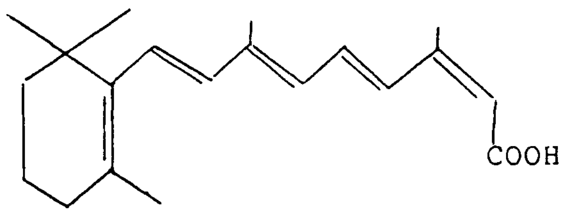

(Ic)

solubilisé dans le même solvant.

2. Procédé de préparation selon la revendication 1, caractérisé par le fait que l'on met en oeuvre un sel d'ammonium quaternaire répondant à la formule (Ia), dans laquelle $R_3$ désigne un groupement benzyle et $R_1$, $R_2$, $R_4$ ont les significations (i) et (ii) définies dans la formule (I).

3. Procédé de préparation selon l'une quelconque des revendications 1 ou 2, caractérisé par le fait que l'on met en oeuvre un sel d'ammonium quaternaire choisi parmi les groupements suivants : N-hexadécyl N,N,N-triméthylammonium, N-benzyl N,N-diméthyl N-hexadécylammonium, N-hexadécyl N,N-diméthyl N,$\beta$-hydroxyéthylammonium, N-dodécyl N-éthyl N,N-diméthylammonium, N-méthyl N-dodécylmorpholinium, N-dodécyl N,N,N-triméthylammonium.

4. Procédé de préparation selon l'une quelconque des revendications 1 à 3, caractérisé par le fait que l'on met en oeuvre un carbonate d'ammonium quaternaire répondant à la formule (Ia).

5. Procédé de préparation selon l'une quelconque des revendications 1 à 4, caractérisé par le fait que le solvant est choisi parmi le méthanol, l'éthanol, l'isopropanol, le tetrahydrofuranne, le dioxane ou leurs mélanges.

6. Procédé de préparation d'une composition destinée à une application topique, caractérisé par le fait que l'on introduit dans un support ou diluant, pharmaceutiquement ou cosmétiquement acceptable, approprié pour une application topique, un composé répondant à la formule (I) :

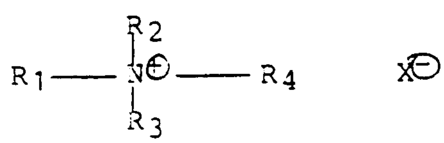

dans laquelle :
$X^{\ominus}$ désigne :
- soit un rétinoate all-trans de formule :

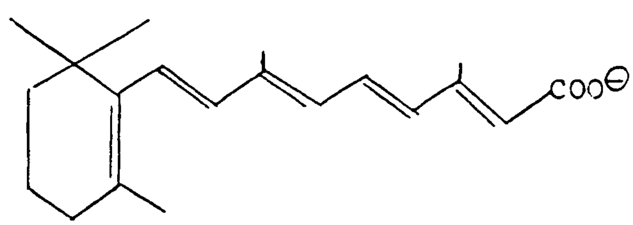

- soit un rétinoate 13-cis de formule :

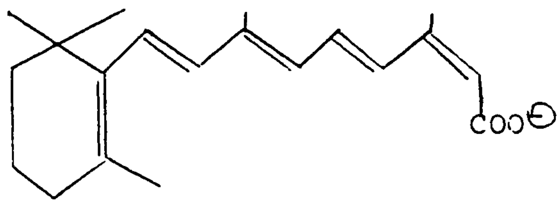

(i) $R_1$, $R_2$, $R_3$, identiques ou différents, désignent un groupement alcoyle linéaire saturé en $C_1$-$C_4$ pouvant porter en bout de chaîne ou dans la chaîne un ou plusieurs groupement(s) hydroxyle(s);

$R_4$ désigne un groupement alcoyle ou alcényle linéaire en $C_{12}$-$C_{18}$;

(ii) $R_3$ désigne le groupement :

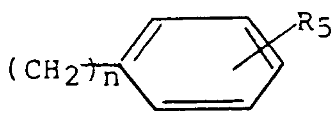

dans lequel n est égal à 0 ou 1, $R_5$ représente un atome d'hydrogène, un hydroxyle, un atome d'halogène, un groupement alcoyle ou un hydroxyalcoyle en $C_1$ à $C_{18}$ ou un groupement acyle en $C_2$ à $C_{18}$;

$R_1$, $R_2$ et $R_4$ ayant les mêmes significations que celles sous (i);

(iii) $R_1$ et $R_2$ peuvent former un hétérocycle aliphatique comportant éventuellement un atome d'oxygène, un autre atome d'azote ou un atome de soufre;

$R_3$ et $R_4$ ayant les mêmes significations que celles sous (i) et (ii).

**7.** Procédé de préparation selon la revendication 6, caractérisé par le fait que dans la formule (I), $R_3$ désigne un groupement benzyle et $R_1$, $R_2$, $R_4$ ont les significations indiquées en (i) et (ii).

**8.** Procédé de préparation selon l'une quelconque des revendications 6 à 7, caractérisé par le fait que le composé répondant à la formule (I) est choisi parmi les composés suivants : rétinoate all-trans de N-hexadécyl N,N,N-triméthylammonium, rétinoate 13-cis de N-hexadécyl N,N,N-triméthylammonium, rétinoate all-trans de N-benzyl N,N-diméthyl N-hexadécylammonium, rétinoate 13-cis de N-benzyl N,N-diméthyl N-hexadécylammonium, rétinoate all-trans de N-hexadécyul N,N-diméthyl N,$\beta$-hydroxyéthylammonium, rétinoate all-trans de N-dodécyl N-éthyl N,N-diméthylammonium, rétinoate all-trans de N-méthyl, N-dodécylmorpholinium, rétinoate all-trans de N-dodécyl N,N,N-triméthylammonium.

**9.** Procédé de préparation d'une composition selon l'une quelconque des revendications 6 à 8, caractérisé par le fait qu'elle est préparée sous forme de solution, d'émulsion, de suspension, de gel ou de dispersion vésiculaire et que le composé de formule (I) y est introduit dans des concentrations comprises entre 0,00001 et 1% en poids par rapport au poids total de la composition.

**10.** Procédé de préparation selon l'une quelconque des revendications 6 à 9, caractérisé par le fait que la composition est préparée sous forme de crème, de lait, de gel, de dispersion, de micro-émulsion, de lotion plus ou moins épaissie, de tampon imbibé, de pommade, de stick ou sous forme de spray ou de mousse et encore sous forme de pain de savon.

**11.** Procédé de préparation selon l'une quelconque des revendications 6 à 10, caractérisé par le fait que l'on introduit en association avec le composé de formule (I), au moins un autre agent antiacnéique choisi parmi les antibactériens, les anti-inflammatoires et les composés antiséborrhéïques.

**12.** Procédé de préparation selon l'une quelconque des revendications 6 à 10, caractérisé par le fait que l'on ajoute un composé choisi parmi le diamino-2,4 pipéridino-6 pyrimidine oxyde-3, le chloro-7 méthyl-3 benzothiadiazine-1,2,4 dioxyde-1,1, le diphényl-5,5 imidazolidine dione-2,4.

**Claims**

**Claims for the following Contracting States : AT, BE, CH, DE, FR, GB, IT, LI, NL, SE**

**1.** Quaternary ammonium retinoate characterised in that it is of the formula:

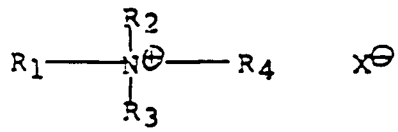

in which:
$X^{\ominus}$ denotes:
- either an all-trans-retinoate of formula:

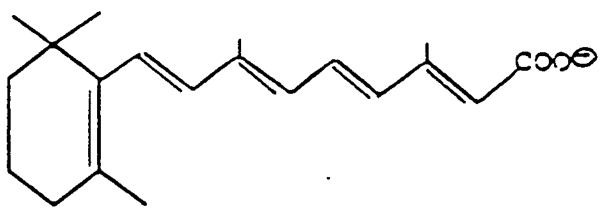

- or a 13-cis-retinoate of formula:

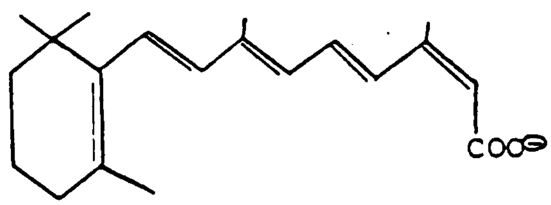

(i) $R_1$, $R_2$ and $R_3$, which are identical or different, denote a $C_1$-$C_4$ saturated linear alkyl group capable of carrying at the end of the chain or inside the chain one or more hydroxyl group or groups;
$R_4$ denotes a $C_{12}$-$C_{18}$ linear alkyl or alkenyl group;
(ii) $R_3$ denotes the group:

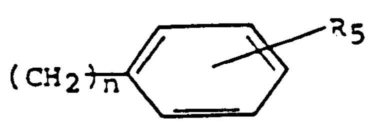

in which n is equal to 0 or 1, $R_5$ represents a hydrogen atom, a hydroxyl, a halogen atom, a $C_1$ to $C_{18}$ alkyl or hydroxyalkyl group or a $C_2$ to $C_{18}$ acyl group;
$R_1$, $R_2$ and $R_4$ having the same meanings as those in (i);
(iii) $R_1$ and $R_2$ may form an aliphatic heterocycle optionally comprising an oxygen atom, another nitrogen atom or a sulphur atom;
$R_3$ and $R_4$ having the same meanings as those in (i) and (ii).

**2.** Compounds according to Claim 1, characterised in that $R_3$ denotes a benzyl group and $R_1$, $R_2$ and $R_4$ have the meanings indicated in (i) and (ii).

**3.** Compounds according to any one of Claims 1 or 2, characterised in that they are chosen from the following compounds: N-hexadecyl-N,N,N-trimethylammonium all-trans-retinoate, N-hexadecyl-N,N,N-trimethylammonium 13-cis-retinoate, N-benzyl-N,N-dimethyl-N-hexadecylammonium all-trans-retinoate, N-benzyl-N,N-dimethyl-N-hexadecyl ammonium 13-cis-retinoate, N-hexadecyl-N,N-dimethyl-N,$\beta$-hydroxyethylammonium all-trans-retinoate, N-dodecyl-N-ethyl-N,N-dimethylammonium all-trans-retinoate,

N-methyl-N-dodecylmorpholinium all-trans-retinoate and N-dodecyl-N,N,N-trimethylammonium all-trans-retinoate.

4. Composition characterised in that it contains in a carrier suitable for a topical application at least one compound such as defined in any one of Claims 1 to 3.

5. Composition according to Claim 4, characterised in that it is provided in the form of a solution, an emulsion, a suspension, a gel or a vesicular dispersion containing at least one compound of formula (I) in concentrations between 0.00001 and 1% by weight relative to the total weight of the composition.

6. Composition according to any one of Claims 4 or 5, characterised in that it is provided in the form of creams, milks, dispersion or micro-emulsion gels, more or less thickened lotions, impregnated pads, pomades, sticks or in the form of a spray or a foam, or alternatively in the form of a soap bar.

7. Composition according to any one of Claims 4 to 6, characterised in that it contains in combination with the compound of formula (I), at least one other antiacne agent chosen from antibacterial agents, anti-inflammatory agents and antiseborrhoeic compounds.

8. Composition according to any one of Claims 4 to 7, characterised in that it contains in addition to the compound of formula (I), 2,4-diamino-6-piperidinopyrimidine 3-oxide, 7-chloro-3-methyl-1,2,4-benzothiadiazine 1,1-dioxide, 5,5-diphenylimidazolidine 2,4-dione.

9. Composition according to any one of Claims 4 to 8 for its therapeutic application.

10. Use of the compound defined in any one of Claims 1 to 3, for the preparation of a composition intended for the treatment of skin disorders.

11. Use according to Claim 10, characterised in that a composition intended for the treatment of acne is prepared.

12. Use of the compound according to any one of Claims 1 to 3 for the preparation of a product which stimulates hair regrowth and/or slows down hair loss.

13. Method of cosmetic treatment characterised in that it consists in applying to the skin a composition according to any one of Claims 4 to 8.

**Claims for the following Contracting States : ES, GR**

1. Process for the preparation of a quaternary ammonium retinoate of the formula (I):

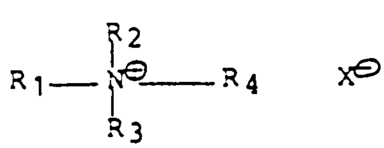

in which:
$X^{\ominus}$ denotes:
- either an all-trans-retinoate of formula:

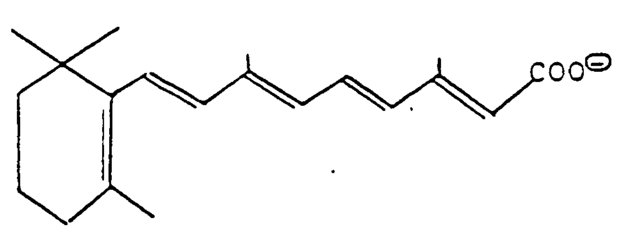

- or a 13-cis-retinoate of formula:

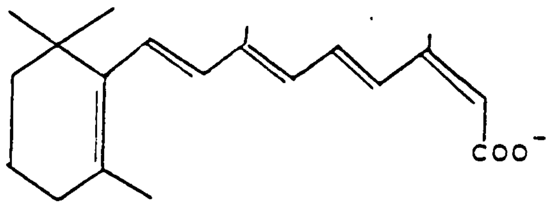

(i) $R_1$, $R_2$ and $R_3$, which are identical or different, denote a $C_1$-$C_4$ saturated linear alkyl group capable of carrying at the end of the chain or inside the chain one or more hydroxyl group or groups;

$R_4$ denotes a $C_{12}$-$C_{18}$ linear alkyl or alkenyl group;

(ii) $R_3$ denotes the group:

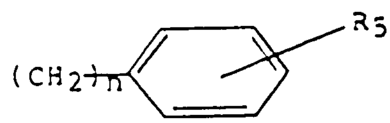

in which n is equal to 0 or 1, $R_5$ represents a hydrogen atom, a hydroxyl, a halogen atom, a $C_1$ to $C_{18}$ alkyl or hydroxyalkyl group or a $C_2$ to $C_{18}$ acyl group;

$R_1$, $R_2$ and $R_4$ having the same meanings as those in (i);

(iii) $R_1$ and $R_2$ may form an aliphatic heterocycle optionally comprising an oxygen atom, another nitrogen atom or a sulphur atom;

$R_3$ and $R_4$ having the same meanings as those in (i) and (ii);

characterised in that a quaternary ammonium salt of the formula (Ia):

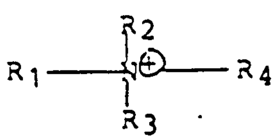

(Ia)

in which $R_1$, $R_2$, $R_3$ and $R_4$ have the same meanings as those defined in formula (I), solubilised in a solvent, is reacted with

- either an all-trans-retinoic acid of formula (Ib):

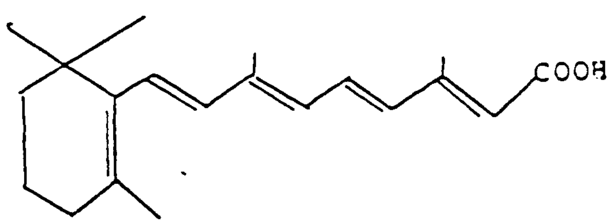

(Ib)

- or a 13-cis-retinoic acid of formula (Ic):

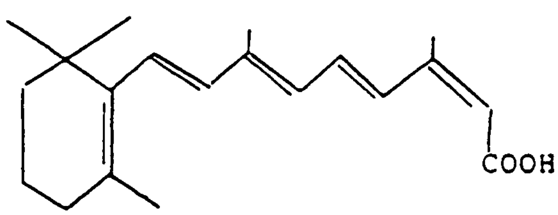

(Ic)

solubilised in the same solvent.

**2.** Preparation process according to Claim 1, characterised in that a quaternary ammonium salt of the formula (Ia) is used, in which $R_3$ denotes a benzyl group and $R_1$, $R_2$ and $R_4$ have the meanings (i) and (ii) defined in formula (I).

**3.** Preparation process according to any one of Claims 1 or 2, characterised in that a quaternary ammonium salt chosen from the following groups: N-hexadecyl-N,N,N-trimethylammonium, N-benzyl-N,N-dimethyl-N-hexadecylammonium, N-hexadecyl-N,N-dimethyl-N,β-hydroxyethylammonium, N-dodecyl-N-ethyl-N,N-dimethylammonium, N-methyl-N-dodecylmorpholinium and N-dodecyl-N,N,N-trimethylammonium is used.

**4.** Preparation process according to any one of Claims 1 to 3, characterised in that a quaternary ammonium carbonate of the formula (Ia) is used.

**5.** Preparation process according to any one of Claims 1 to 4, characterised in that the solvent is chosen from methanol, ethanol, isopropanol, tetrahydrofuran, dioxane or their mixtures.

**6.** Process for the preparation of a composition intended for a topical application, characterised in that there is introduced into a pharmaceutically or cosmetically acceptable carrier or diluent, suitable for a topical application, a compound of the formula (I):

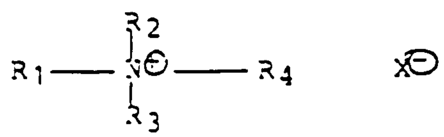

in which:
$X^{\ominus}$ denotes:
- either an all-trans-retinoate of formula:

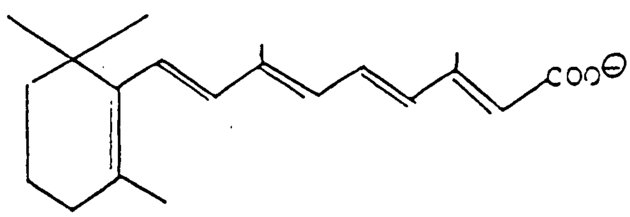

- or a 13-cis-retinoate of formula:

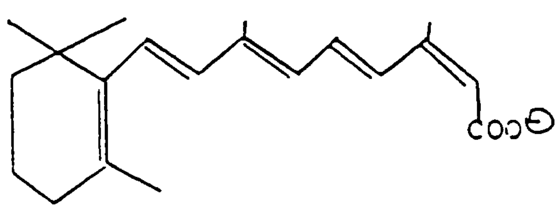

(i) $R_1$, $R_2$ and $R_3$, which are identical or different, denote a $C_1$-$C_4$ saturated linear alkyl group capable of carrying at the end of the chain or inside the chain one or more hydroxyl group or groups;
$R_4$ denotes a $C_{12}$-$C_{18}$ linear alkyl or alkenyl group;
(ii) $R_3$ denotes the group:

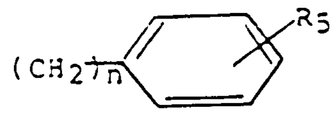

in which n is equal to 0 or 1, $R_5$ represents a hydrogen atom, a hydroxyl, a halogen atom, a $C_1$ to $C_{18}$ alkyl or hydroxyalkyl group or a $C_2$ to $C_{18}$ acyl group;

$R_1$, $R_2$ and $R_4$ having the same meanings as those in (i);

(iii) $R_1$ and $R_2$ may form an aliphatic heterocycle optionally comprising an oxygen atom, another nitrogen atom or a sulphur atom;

$R_3$ and $R_4$ having the same meanings as those in (i) and (ii).

**7.** Preparation process according to Claim 6, characterised in that in formula (I), $R_3$ denotes a benzyl group and $R_1$, $R_2$ and $R_4$ have the meanings indicated in (i) and (ii).

**8.** Preparation process according to any one of Claims 6 to 7, characterised in that the compound of formula (I) is chosen from the following compounds: N-hexadecyl-N,N,N-trimethylammonium all-trans-retinoate, N-hexadecyl-N,N,N-trimethylammonium 13-cis-retinoate, N-benzyl-N,N-dimethyl-N-hexadecylammonium all-trans-retinoate, N-benzyl-N,N-dimethyl-N-hexadecyl ammonium 13-cis-retinoate, N-hexadecyl-N,N-dimethyl-N,$\beta$-hydroxyethylammonium all-trans-retinoate, N-dodecyl-N-ethyl-N,N-dimethylammonium all-trans-retinoate, N-methyl-N-dodecylmorpholinium all-trans-retinoate and N-dodecyl-N,N,N-trimethylammonium all-trans-retinoate.

**9.** Process for the preparation of a composition according to any one of Claims 6 to 8, characterised in that it is prepared in the form of a solution, an emulsion, a suspension, a gel, a vesicular dispersion and in that the compound of formula (I) is introduced therein in concentrations between 0.00001 and 1% by weight relative to the total weight of the composition.

**10.** Preparation process according to any one of Claims 6 to 9, characterised in that the composition is prepared in the form of a cream, a milk, a gel, a dispersion, a micro-emulsion, a more or less thickened lotion, an impregnated pad, a pomade, a stick or in the form of a spray or a foam and also in the form of a soap bar.

**11.** Preparation process according to any one of Claims 6 to 10, characterised in that at least one other antiacne agent chosen from antibacterial agents, anti-inflammatory agents and antiseborrhoeic compounds, is introduced in combination with the compound of formula (I).

**12.** Preparation process according to any one of Claims 6 to 10, characterised in that a compound chosen from 2,4-diamino-6-piperidinopyrimidine 3-oxide, 7-chloro-3-methyl-1,2,4-benzothiadiazine 1,1-dioxide and 5,5-diphenylimidazolidine 2,4-dione is added.

**Patentansprüche**

**Patentansprüche für folgende Vertragsstaaten : AT, BE, CH, DE, FR, GB, IT, LI, NL, SE**

**1.** Quaternäres Ammonium-Retinoat der Formel:

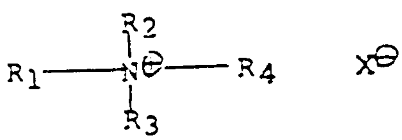

worin $X^{\ominus}$

- entweder ein Retinoat-all-trans der Formel:

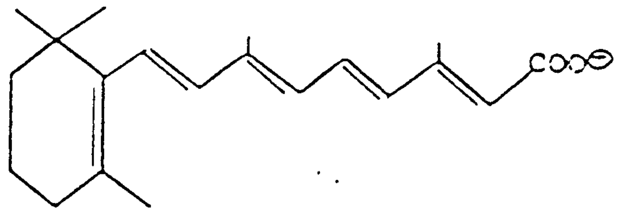

- oder ein Retinoat-13-cis der Formel:

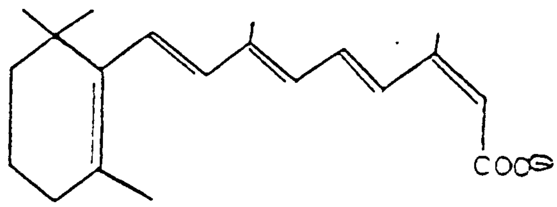

darstellt, wobei
(i) $R_1$, $R_2$, $R_3$, gleich oder verschieden, eine lineare gesättigte $C_1$-$C_4$-Alkoylgruppe darstellen, welche am Ende oder in der Kette eine oder mehrere Hydroxylgruppe(n) aufweisen können;
$R_4$ eine lineare $C_{12}$-$C_{18}$-Alkoyl- oder -Alkenylgruppe darstellt;
(ii) $R_3$ die Gruppe:

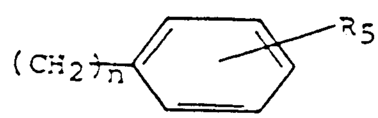

dartellt, worin n 0 oder 1 ist, $R_5$ ein Wasserstoffatom, Hydroxyl, ein Halogenatom, eine $C_1$-$C_{18}$-Alkoyl- oder -Hydroxyalkoylgruppe oder eine $C_2$-$C_{18}$-Acylgruppe darstellt;
$R_1$, $R_2$ und $R_4$ dieselben Bedeutungen wie unter (i) haben;
(iii) $R_1$ und $R_2$ einen aliphatischen Heterozyklus bilden können, der gegebenenfalls ein Sauerstoffatom, ein weiteres Stickstoffatom oder ein Schwefelatom aufweist;
$R_3$ und $R_4$ dieselben Bedeutungen wie unter (i) und (ii) haben.

**2.** Verbindungen gemäß Anspruch 1, dadurch **gekennzeichnet,** daß $R_3$ eine Benzylgruppe darstellt und $R_1$, $R_2$ und $R_4$ die unter (i) und (ii) aufgeführten Bedeutungen haben.

**3.** Verbindungen gemäß eines jeden der Ansprüche 1 oder 2, dadurch **gekennzeichnet,** daß sie unter den folgenden Verbindungen ausgewählt sind:
N-Hexadecyl-N,N,N-trimethylammonium-Retinoat-all-trans, N-Hexadecyl-N,N,N-trimethylammonium-Retinoat-13-cis, N-Benzyl-N,N-dimethyl-N-hexadecylammonium-Retinoat-all-trans, N-Benzyl-N,N-dimehtyl-N,hexadecylammonium-Retinoat-13-cis, N-Hexadecy-N,N-dimethyl-N,β-hydroxyethylammonium-Retinoat-all-trans, N-Dodecyl-N-ethyl-N,N-dimethylammonium-Retinoat-all-trans, N-Methyl-N-dodecylmorpholinium-Retinoat-all-trans, N-Dodecyl-N,N,N-trimethylammonium-Retinoat-all-trans.

**4.** Mittel, dadurch **gekennzeichnet,** daß es in einem zur topischen Anwendung geeigneten Träger mindestens eine Verbindung gemäß eines jeden der Ansprüche 1 bis 3 enthält.

**5.** Mittel gemäß Anspruch 4,
dadurch **gekennzeichnet,** daß
es in Form einer Lösung, Emulsion, Suspension, eines Gels oder einer Schaumdispersion vorliegt, welche mindestens eine Verbindung der Formel (I) in Konzentrationen von 0,00001 bis 1 Gew.%, bezogen auf Gesamtgewicht des Mittels, enthalten.

**6.** Mittel gemäß eines jeden der Ansprüche 4 oder 5,
dadurch **gekennzeichnet,** daß
es in Form von Crèmeprodukten, Milchprodukten, Dispersions- oder Mikroemulsionsgelen, mehr oder weniger verdickten Lotionen, getränkten Tampons, Pomaden, Stiften oder in Form eines Sprays oder Schaums oder auch in Form eines Stücks Seife vorliegt.

**7.** Mittel gemäß eines jeden der Ansprüche 4 bis 6,
dadurch **gekennzeichnet,** daß
es zusammen mit der Verbindung der Formel (I) mindestens ein weiteres Antiaknemittel enthält, ausgewählt unter Antibakterien-, Antientzündungsmitteln und antiseborrheischen Verbindungen.

**8.** Mittel gemäß eines jeden der Ansprüche 4 bis 7,
dadurch **gekennzeichnet,** daß
es zusätzlich zur Verbindung der Formel (I) Diamino-2,4-piperidino-6-pyrimidin-oxid-3, Chlor-7-methyl-3-benzothiadiazin-1,2,4-dioxid-1,1, Diphenyl-5,5-imidazolidin-dion-2,4 enthält.

**9.** Mittel gemäß eines jeden der Ansprüche 4 bis 8 zur therapeutischen Anwendung.

**10.** Verwendung der Verbindung gemäß eines jeden der Ansprüche 1 bis 3 zur Herstellung eines Mittels zur Behandlung von Hautaffekten.

**11.** Verwendung gemäß Anspruch 10,
dadurch **gekennzeichnet,** daß
man ein Mittel zur Behandlung von Akne herstellt.

**12.** Verwendung der Verbindung gemäß eines jeden der Ansprüche 1 bis 3 zur Herstellung eines Produkts, das das Wachstum und/oder die Verminderung des Ausfalls der Haare stimuliert.

**13.** Kosmetisches Behandlungsverfahren,
dadurch **gekennzeichnet,** daß
man ein Mittel gemäß eines jeden der Ansprüche 4 bis 8 auf die Haut aufträgt.

**Patentansprüche für folgende Vertragsstaaten : ES, GR**

**1.** Verfahren zur Herstellung eines quaternären Ammonium-Retinoats der Formel (I):

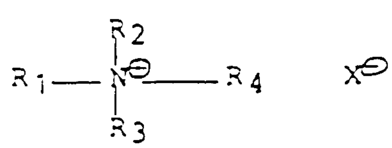

worin $X^{\ominus}$
- entweder ein Retinoat-all-trans der Formel:

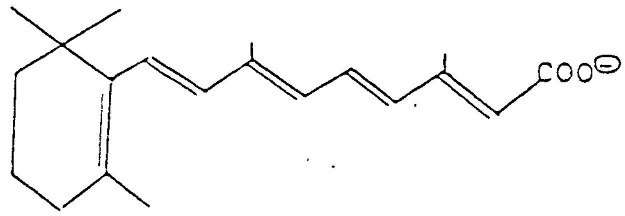

- oder ein Retinoat-13-cis der Formel:

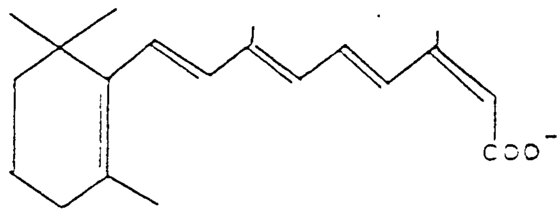

darstellt, wobei
(i) $R_1$, $R_2$, $R_3$, gleich oder verschieden, eine lineare gesättigte $C_1$-$C_4$-Alkoylgruppe darstellen, welche am Ende oder in der Kette eine oder mehrere Hydroxylgruppe(n) aufweisen können;
$R_4$ eine lineare $C_{12}$-$C_{18}$-Alkoyl- oder -Alkenylgruppe darstellt;
(ii) $R_3$ die Gruppe:

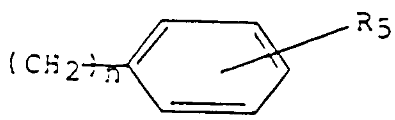

dartellt, worin n 0 oder 1 ist, $R_5$ ein Wasserstoffatom, Hydroxyl, ein Halogenatom, eine $C_1$-$C_{18}$-Alkoyl- oder -Hydroxyalkoylgruppe oder eine $C_2$-$C_{18}$-Acylgruppe darstellt;
$R_1$, $R_2$ und $R_4$ dieselben Bedeutungen wie unter (i) haben;
(iii) $R_1$ und $R_2$ einen aliphatischen Heterozyklus bilden können, der gegebenenfalls ein Sauerstoffatom, ein weiteres Stickstoffatom oder ein Schwefelatom aufweist;
$R_3$ und $R_4$ dieselben Bedeutungen wie unter (i) und (ii) haben,

dadurch **gekennzeichnet,** daß man ein quaternäres Ammoniumsalz der Formel (Ia):

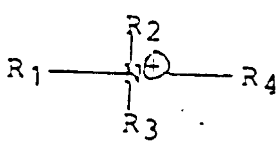

(Ia)

worin $R_1$, $R_2$, $R_3$ und $R_4$ dieselben Bedeutungen wie in der Formel (I) haben, welches in einem Lösungsmittel gelöst ist, mit

- entweder einer Retinoesäure-all-trans der Formel (Ib)

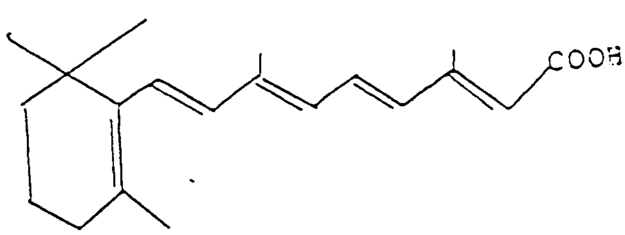

(Ib)

- oder einer Retinoesäure-13-cis der Formel (Ic):

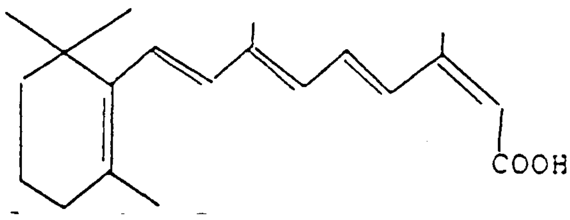

(Ic)

welche in demselben Lösungsmittel gelöst sind, reagieren läßt.

**2.** Verfahren gemäß Anspruch 1,
dadurch **gekennzeichnet,** daß
man ein quaternäres Ammoniumsalz der Formel (Ia) einsetzt, worin $R_3$ eine Benzylgruppe darstellt, und $R_1$, $R_2$ und $R_4$ die unter (i) und (ii) für die Formel (i) definierten Bedeutungen haben.

**3.** Verfahren gemäß eines jeden der Ansprüche 1 oder 2,
dadurch **gekennzeichnet,** daß
man ein quaternäres Ammoniumsalz einsetzt, ausgewählt unter den folgenden Gruppen:
N-Hexadecyl, N,N,N-Trimethylammonium, N-Benzyl, N,N-Dimethyl-N-hexadecylammonium, N-Hexadecyl-N,N-dimethyl-N,$\beta$-hydroxyethylammonium, N-Dodecyl, N-Ethyl, N,N-Dimethylammonium, N-Methyl-N-dodecylmorpholinium, N-Dodecyl-N,N-N-trimethylammonium.

**4.** Verfahren gemäß eines jeden der Ansprüche 1 bis 3,
dadurch **gekennzeichnet,** daß
man ein quaternäres Ammoniumcarbonat der Formel (Ia) einsetzt.

**5.** Verfahren gemäß eines jeden der Ansprüche 1 bis 4,
dadurch **gekennzeichnet,** daß
das Lösungsmittel unter Methanol, Ethanol, Isopropanol, Tetrahydrofuran, Dioxan oder deren Mischungen ausgewählt ist.

**6.** Verfahren zur Herstellung eines Mittels zur topischen Anwendung,
dadurch **gekennzeichnet,** daß
man in ein zur topischen Anwendung geeignetes, pharmazeutisch oder kosmetisch verträgliches Träger- oder Verdünnungsmittel eine Verbindung der Formel (I) einbringt:

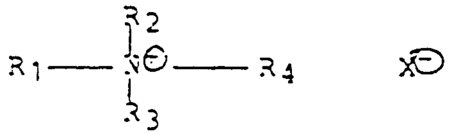

worin $X^{\ominus}$

- entweder ein Retinoat-all-trans der Formel:

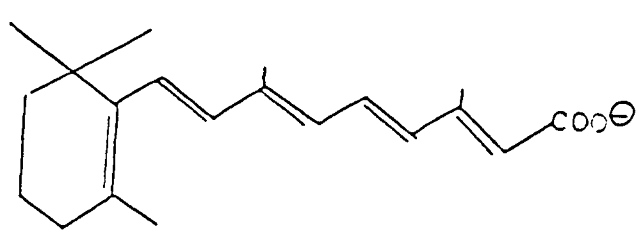

- oder ein Retinoat-13-cis der Formel:

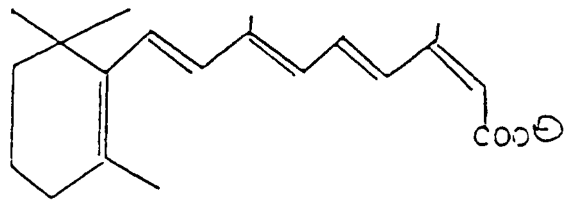

darstellt, wobei

(i) $R_1$, $R_2$, $R_3$, gleich oder verschieden, eine lineare gesättigte $C_1$-$C_4$-Alkoylgruppe darstellen, welche am Ende oder in der Kette eine oder mehrere Hydroxylgruppe(n) aufweisen können;
$R_4$ eine lineare $C_{12}$-$C_{18}$-Alkoyl- oder -Alkenylgruppe darstellt;
(ii) $R_3$ die Gruppe:

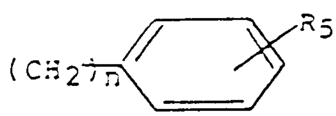

dartellt, worin n 0 oder 1 ist, $R_5$ ein Wasserstoffatom, Hydroxyl, ein Halogenatom, eine $C_1$-$C_{18}$-Alkoyl- oder -Hydroxyalkoylgruppe oder eine $C_2$-$C_{18}$-Acylgruppe darstellt;
$R_1$, $R_2$ und $R_4$ dieselben Bedeutungen wie unter (i) haben;
(iii) $R_1$ und $R_2$ einen aliphatischen Heterozyklus bilden können, der gegebenenfalls ein Sauerstoffatom, ein weiteres Stickstoffatom oder ein Schwefelatom aufweist;
$R_3$ und $R_4$ dieselben Bedeutungen wie unter (i) und (ii) haben.

**7.** Verfahren nach Anspruch 6,
dadurch **gekennzeichnet,** daß in
Formel (I) $R_3$ eine Benzylgruppe darstellt und $R_1$, $R_2$ und $R_4$ die unter (i) und (ii) angegebenen Bedeutungen haben.

**8.** Verfahren gemäß eines jeden der Ansprüche 6 bis 7,
dadurch **gekennzeichnet,** daß
die Verbindung der Formel (I) unter den folgenden Verbindungen ausgewählt ist:
N-Hexadecyl-N,N,N-trimethylammonium-Retinoat-all-trans, N-Hexadecyl-N,N,N-trimethylammonium-Retinoat-13-cis, N-Benzyl-N,N-dimethyl-N-hexadecylammonium-Retinoat-all-trans, N-Benzyl-N,N-dimehtyl-N,hexadecylammonium-Retinoat-13-cis, N-Hexadecy-N,N-dimethyl-N,β-hydroxyethylammonium-Retinoat-all-trans, N-Dodecyl-N-ethyl-N,N-dimethyl-ammonium-Retinoat-all-trans, N-Methyl-N-dodecylmorpholinium-Retinoat-all-trans, N-Dodecyl-N,N,N-trimethylammonium-Retinoat-all-trans.

**9.** Verfahren zur Herstellung eines Mittels gemäß eines jeden der Ansprüche 6 bis 8,
dadurch **gekennzeichnet,** daß
man es in Form einer Lösung, Emulsion, Suspension, eines Gels oder einer Schaumdispersion herstellt und daß die Verbindung der Formel (I) in Konzentrationen zwischen 0,00001 bis 1 Gew.%, bezogen auf Gesamtgewicht des Mittels, eingebracht wird.

**10.** Verfahren gemäß eines jeden der Ansprüche 6 bis 9,
dadurch **gekennzeichnet,** daß
das Mittel in Form einer Crème, Milch, eines Gels, einer Dispersion, Mikroemulsion, mehr oder weniger verdickten Lotion, eines getränkten Tampons, einer Pomade, eines Stifts oder in Form eines Sprays oder Schaums oder auch in Form eines Stücks Seife hergestellt wird.

**11.** Verfahren gemäß eines jeden der Ansprüche 6 bis 10,
dadurch **gekennzeichnet,** daß
man zusammen mit der Verbindung der Formel (I) mindestens ein weiteres Antiaknemittel einbringt, ausgewählt unter Antibakterien-, Antientzündungsmitteln und antiseborrheischen Verbindungen.

**12.** Verfahren gemäß eines jeden der Ansprüche 6 bis 10, dadurch **gekennzeichnet,** daß man eine Verbindung zufügt, ausgewählt unter Diamino-2,4-piperidino-6-pyrimidin-oxid-3, Chlor-7-methyl-3-benzothiadiazin-1,2,4-dioxid-1,3, Diphenyl-5,5-imidazolidin-dion-2,4.